# FASCICULE DE BREVET EUROPEEN

(11) **EP 0 801 046 B1**
(45) Date de publication et mention de la délivrance du brevet: **19.12.2001**
(21) Numéro de dépôt: 97400731.2
(22) Date de dépôt: 28.03.1997
(51) Int. Cl.: C07C 11/02, C07C 1/20

(54) **Procédé de production d'oléfine(s) tertiaire(s) par décomposition de l'éther correspondant à l'aide d'un catalyseur particulier**
Verfahren zur Herstellung von (eines) tertiären Olefin(en) unter Verwendung eines bestimmten Katalysators
Process for the preparation of (a) tertiary olefin(s) by decomposition of the corresponding ether using a particular catalyst

(30) Priorité: 09.04.1996 FR 9604418
(43) Date de publication de la demande: 15.10.1997
(73) Titulaire: INSTITUT FRANCAIS DU PETROLE, 92502 Rueil-Malmaison (FR)
(72) Inventeur: Marion, Marie-Claire, 69100 Villeurbanne (FR); Forestiere, Alain, 69390 Vernaison (FR); Barret, Armand, 69390 Vernaison (FR)

(56) Documents cités:
- DE-A- 3 509 292
- FR-A- 2 669 021
- GB-A- 2 188 853
- US-A- 5 354 831

## Description

L'invention concerne un procédé de décomposition d'éther(s) alkyl tertiaire(s), pour la production d'oléfine(s) tertiaire(s) de haute pureté, caractérisé par l'utilisation d'un catalyseur comprenant au moins un solide de type polysiloxane, greffé par au moins un groupement organique de type acide alkyl sulfonique, aryl sulfonique ou alkyaryl sulfonique. Le procédé de l'invention est mis en oeuvre dans des conditions opératoires spécifiques de manière à garantir une activité et une stabilité satisfaisantes dudit catalyseur.

Il est connu que les oléfines possédant un atome de carbone tertiaire prenant part à une double liaison subissent une réaction d'addition sélective avec les alcools pour former des tertio alkyl éthers. Cette addition se fait par un processus exothermique et est catalysé par les acides en général. Ces réactions sont exploitées pour la synthèse des produits oxygénés à haut indice d'octane, tels que le MTBE (Méthyl Tertio Butyl Ether), l'ETBE (Ethyl Tertio Butyl Ether), le TAME (Tertio Amyl Ether), l'ETAE (Ethyl Tertio Amyl Ether). Pour obtenir le TAME et l'ETAE, on part généralement d'un mélange contenant deux isoamylènes que sont le méthyl-2 butène-1 et le méthyl-2 butène-2 (le méthyl-3 butène-1 étant peu réactif). On peut aussi exploiter la sélectivité de ces réactions de synthèse d'éthers pour séparer les oléfines tertiaires des hydrocarbures dans lesquels elles sont contenues. En effet, la séparation de certaines oléfines entre elles, par exemple la séparation de l'isobutène et du butène-1 est difficile par un processus de distillation classique. Par contre, la séparation de l'éther alkyl tertiaire (qui est produit de façon sélective), de la coupe d'hydrocarbures dont il est issu, est généralement aisée. Une fois isolé, l'éther peut être décomposé à nouveau pour former l'oléfine tertiaire de départ et l'alcool utilisé. Cela a lieu par un processus endothermique, en présence d'un catalyseur généralement acide et à une température plus élevée que pour la synthèse. L'oléfine tertiaire produite peut alors être de pureté élevée, en fonction des conditions d'optimisation. .

Par rapport à d'autres méthodes permettant de produire des oléfines tertiaires de haute pureté telles que celles utilisant des réactions d'isomérisation, dans des procédés mettant en oeuvre de la distillation réactive (par exemple la séparation isobutène/butène par transformation du butène-1 en butène-2), le schéma incorporant la synthèse puis la décomposition de l'éther bénéficie de toute l'infrastructure relative à l'importance croissance des éthers dans les essences reformulées. De nombreuses raffineries, partout dans le monde, possèdent des installations de productions d'éthers purs tel que le MTBE.

D'autre part, il y a également une circulation importante d'éthers purs tel que le MTBE sur le marché international. Cela signifie que la production d'oléfine tertiaire, par exemple d'isobutène, de haute pureté, à partir d'éther, par exemple de MTBE, peut être mise en oeuvre aisément partout dans le monde, y compris en dehors des raffineries où ces éthers sont généralement produits.

L'exploitation de la sélectivité des réactions de décomposition des éthers tertio alkyls en oléfines tertiaires correspondantes est connue depuis longtemps, comme le montre par exemple la demande de brevet EP-A-0.068.785 (SUMITOMO) et divers solides acides ont été décrits comme catalyseurs de ces réactions. Ainsi, la demande de brevet FR-A-2 291.958 (SNAMPROGETTI) décrit l'utilisation des sels, oxydes ou complexes de l'uranium tétravalent, pouvant être supporté sur une alumine alpha par exemple, présentant une acidité de Lewis. Le brevet US-A-4,656,016 (SNAMPROGETTI) décrit l'utilisation de silice modifiée par l'introduction de bore au sein de son réseau et éventuellement par des cations (H⁺, NH₄⁺ ou cation métallique). La demande de brevet WO-A-91/01.804 (EXXON) décrit l'utilisation d'argile (montmorillonite, kaolinite, attapulgite, bentonite...). Enfin, le brevet US-A-5.095.164 décrit l'utilisation de résines échangeuses d'ions par exemple les résines styrène-divinylbenzène sulphonées (qui sont d'ailleurs utilisées généralement dans les procédés de synthèse des tertio alkyl éthers). On peut citer ainsi la résine Amberlyst 15 de ROHM & HAAS ou la résine M-31 commercialisée par DOW CHEMICAL.

Un des inconvénients majeurs des résines précédemment citées est l'impossibilité de les utiliser à haute température, plus précisément généralement au-dessus de 120 °C. En effet, à haute température, ces résines se désulfonent et perdent donc ainsi leur activité et/ou acidité. Or les réactions de décomposition des éthers sont endothermiques ; l'équilibre thermodynamique de la réaction est donc d'autant plus déplacé vers la production de l'oléfine que la température est haute. Une température d'opération limitée à 120 °C se traduit par une conversion de l'éther faible et limitée par les lois de la thermodynamique.

Le brevet US-A-5.095.164 décrit un procédé de décomposition des tertio-alkyl éthers en présence par exemple de résines macroporeuses styrène-divinylbenzène sulphonées mettant en oeuvre un appareillage de distillation. Le catalyseur est placé en fond de colonne et il opère entre 50 et 100 °C, de préférence entre 60 et 80 °C. L'équilibre thermodynamique de la réaction de décomposition, mal placé à cause de la température d'opération peu élevée, est déplacé par élimination des produits de la réaction (oléfine tertiaire et alcool correspondant) par distillation. Toutefois, un tel procédé présente des difficultés pour la purification des produits. Il met notamment en oeuvre des quantités d'eau importantes pour la récupération de l'alcool. De plus, l'éther non converti est récupéré en fond de colonne avec des quantités d'alcool non négligeables. Il doit alors être purifié avant d'être recyclé au procédé.

Par ailleurs, d'autres solides catalytiques, par exemple à base d'alumine, silice ou silice-alumine, nécessitent l'adjonction d'eau de façon à améliorer la récupération de l'alcool, et éviter la réaction secondaire de formation du dialkyl éther correspondant, qui est par exemple dans le cas du méthanol : 2 MeOH <----> Me-O-Me (=DME=Di Méthyl Ether) + H2O.

Ceci est décrit notamment dans les demandes de brevet GB-A-1.165.479 (SHELL) et EP-A-0.589.557 (SUMITOMO). Toutefois, la présence d'eau fait baisser l'activité du catalyseur, par abaissement de son acidité (voir en particulier la demande de brevet GB-A-1.165.479) et peut alors contraindre à opérer à plus haute température, ce qui peut nuire à la durée de vie du catalyseur. De plus, la présence d'eau induit une réaction secondaire supplémentaire : l'eau réagit avec l'oléfine tertiaire produite pour former un alcool, comme par exemple dans le cas de la décomposition du MTBE (ou de l'ETBE) : isobutène + H2O ---> ABT (= Alcool Tertio Butylique ou méthyl-2 propanol-2). Selon ce processus, on enregistre donc une perte du rendement dans l'oléfine tertiaire souhaitée.

De façon générale, les procédés de décomposition des éthers -connus de l'homme du métier mettent en oeuvre des catalyseurs présentant au moins un des inconvénients suivants : faible activité ou faible sélectivité ou faible stabilité dans le temps. C'est ainsi par exemple que le procédé décrit .par EXXON (demande de brevet WO-A-91/01.804), et utilisant un catalyseur à base d'argile, propose un système permettant de régénérer le catalyseur in situ. On peut citer également le brevet de la demanderesse US-A-5.171.920 qui met en oeuvre un catalyseur à base de silice modifié par l'addition d'au moins un élément tel que Li, Cs, Mg, Ca ou La par exemple. De tels solides ne sont pas très actifs par manque d'acidité, et ils présentent une stabilité médiocre dans le temps : les données du tableau 1 de l'exemple 13 dudit brevet indiquent qu'en 800 heures, il est nécessaire d'augmenter la température de 50 °C pour maintenir constant le niveau de conversion de l'éther.

Le procédé selon l'invention permet de remédier aux inconvénients précités. Il présente l'originalité d'utiliser un catalyseur comprenant au moins un solide de type polysiloxane, greffé par au moins un groupement organique de type acide alkyl sulfonique, aryl sulfonique, ou alkyaryl sulfonique, lequel catalyseur présente de nombreux avantages.

L'invention concerne un procédé de décomposition d'éther(s) alkyl tertiaire(s), de préférence choisi dans le groupe formé par l'ETBE, MTBE, le TAME et l'ETAE, pour la production d'oléfine(s) tertiaire(s), de préférence d'isobutène ou d'isoamylènes (méthyl-2 butène-1 et méthyl-2 butène-2), généralement de haute pureté, mettant en oeuvre un catalyseur comprenant au moins un solide de type polysiloxane, greffé par au moins un groupement organique de type acide alkyl sulfonique, aryl sulfonique, ou alkyaryl sulfonique, ledit procédé étant mis en oeuvre dans des conditions opératoires telles que la pression (relative) est comprise entre 1 et 10.10⁵ Pa, la température est comprise entre 100 et 250 °C et la VVH (vitesse spatiale liquide horaire, exprimée en volume du charge liquide par volume de catalyseur et par heure) est comprise entre 4,3 et 25 h⁻¹.

Un solide de type polysiloxane greffé par au moins un groupement organique de type acide alkyl sulfonique, aryl sulfonique, ou alkyaryl sulfonique, compris dans le catalyseur utilisé dans le procédé selon l'invention, est en particulier commercialisé par la société DEGUSSA, sous la marque déposée "DELOXAN". La préparation d'un tel solide est décrite dans les brevets US-A-4.552.700 , US-A-5.354.831 et US-A-5.380.791. Ces solides présentent une acidité de Brönsted forte. La possibilité de l'utilisation de tels solides dans des réactions de décomposition d'éthers est simplement mentionnée dans le texte du brevet US-A-5.354.831 (voir colonne 9 lignes 43 à 46), sans qu'elle fasse l'objet d'autres développements. Une des mises en oeuvre préférée du procédé selon l'invention est donc telle que ledit catalyseur comprend du DELOXAN.

De préférence, le solide de type polysiloxane, greffé par au moins un groupement organique de type acide alkyl sulfonique, aryl sulfonique, ou alkyaryl sulfonique compris dans le catalyseur utilisé dans le procédé selon l'invention comprend généralement au moins une unité de formule (1) suivante :

(O_{3/2}Si--R¹--SO₃⁻) H⁺ (1),

où R¹ est un radical alkyl, aryl ou alkylaryl.

De façon surprenante, le procédé selon l'invention permet d'obtenir des performances catalytiques très améliorées par rapport aux catalyseurs de l'art antérieur, et étonnamment supérieures à ce que l'homme du métier aurait pu envisager à la vue des trois brevets cités ci-dessus.

Ainsi, les catalyseurs comportant au moins un solide de type polysiloxane, greffé par au moins un groupement organique de type acide alkyl sulfonique, aryl sulfonique, ou alkyaryl sulfonique présentent de nombreux avantages lors de leur utilisation dans le procédé selon l'invention.

Un des avantages du procédé selon l'invention est que de tels catalyseurs sont très actifs pour la réaction de décomposition d'éthers tertio alkyl en oléfines tertiaires correspondantes, par rapport à ce qui est actuellement décrit dans la littérature, ce qui permet d'opérer à basse température, par exemple 130 °C, ce qui favorise la bonne stabilité du catalyseur, et qui peut se traduire également par une durée de vie du catalyseur élevée.

Un autre des avantages du procédé selon l'invention est que, compte tenu de l'endothermicité de la réaction, et donc du déplacement favorable de l'équilibre thermodynamique par une élévation de température, l'utilisation de tels catalyseurs permet généralement de trouver un intérêt à travailler à haute température (typiquement entre 180 à 220 °C), bien que l'utilisation de telles températures ne soit pas obligatoire dans le cadre de la présente invention. Le fait que le solide greffé soit minéral et non organique, et de type polysiloxane, permet de travailler à de telles températures sans dégradation notable du catalyseur. Dans le cas de l'utilisation de hautes températures dans le procédé selon l'invention, la forte activité des catalyseurs utilisés selon le procédé de la présente invention nous autorise à travailler avec de fortes VVH (vitesse spatiale liquide horaire, exprimée en volume de charge liquide par volume de catalyseur et par heure). Ceci se traduit par la réduction du volume catalytique nécessaire et également par la diminution des équipements de l'installation (d'où un double intérêt économique). En outre, la possibilité de travailler avec des vitesses de passage importantes (VVH élevée) diminue la part des réactions secondaires : on obtient finalement de très bons rendements en oléfine et en alcool.

Enfin, un autre des avantages du procédé selon l'invention est que de tels catalyseurs présentent une excellente stabilité dans le temps pour la réaction de décomposition d'éthers tertio alkyle en oléfine(s) tertiaire(s) correspondante, ce qui à la fois facilite les opérations et ce qui présente un avantage économique certain, basé sur des améliorations telles que des périodes d'arrêt moins fréquentes et une économie globale sur le coût des catalyseurs.

Les conditions opératoires du procédé selon l'invention sont généralement les suivantes. La pression (relative) est généralement comprise entre 1 et 10.10⁵ Pa, de préférence entre 1 et 7.10⁵ Pa. L'utilisation de telles pression permet généralement l'utilisation de systèmes simples de refroidissement à eau pour la récupération de l'oléfine. Dans le cas particulier, préféré selon l'invention, de l'obtention de l'isobutène à partir de MTBE ou d'ETBE, la pression est généralement comprise entre 5 et 10.10⁵ Pa, de préférence 5 et 7.10⁵ Pa. Dans le cas particulier de l'obtention des isoamylènes (par décomposition du TAME ou de l'ETAE), la pression du procédé est généralement comprise entre 1 et 5.10⁵ Pa, de préférence entre 1 et 3.10⁵ Pa. La température est généralement comprise entre 100 et 250°C, de préférence entre 120 et 220°C, et de façon encore plus préférée entre 160 et 220°C. La VVH, définie précédemment, est comprise entre 4,3 et 25 h⁻¹ ; la VVH est un paramètre en général directement lié à la température opératoire, de façon à ce que la combinaison, entre autres, de ces deux paramètres permette d'optimiser le rendement des produits (conversion de l'éther optimale, tout en maintenant de bonnes sélectivités vis-à-vis des produits, oléfine tertiaire et alcool, c'est-à-dire en minimisant les réactions secondaires, ainsi qu'il est connu de l'homme du métier).

Le procédé selon l'invention est particulièrement bien adapté pour l'obtention d'oléfines tertiaires, généralement de haute pureté, de formule 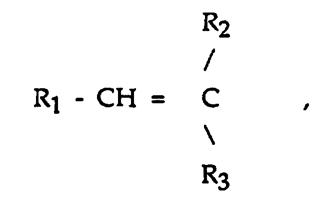 à partir des éthers correspondants de formule 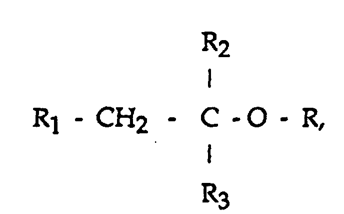 où R1 est chacun choisi dans le groupe formé par l'atome d'hydrogène, les radicaux alkyles par exemple les radicaux méthyle, éthyle, n-propyle et isopropyle et R, R2 et R3,, identiques ou différents, sont chacun choisis dans le groupe formé par les radicaux alkyles par exemple les radicaux méthyle, éthyle, n-propyle et isopropyle.

L'alcool primaire (R-OH) récupéré après décomposition de l'éther contient de préférence de 1 à 6 atomes de carbone par molécule.

Le procédé selon l'invention peut notamment s'appliquer à la décomposition du MTBE ou de l'ETBE, en vue de l'obtention d'isobutène de haute pureté (et de méthanol ou d'éthanol).

Le procédé selon l'invention peut aussi s'appliquer à la décomposition du TAME ou de l'ETAE, en vue de l'obtention d'isoamylènes de haute pureté (et de méthanol ou d'éthanol).

Le procédé selon l'invention est généralement mis en oeuvre dans au moins une zone réactionnelle comprenant au moins un réacteur, chaque réacteur fonctionnant soit en lit fixe, soit en lit mobile, soit en lit expansé ou encore en lit fluidisé. Il est possible de combiner les différents modes de fonctionnement de réacteur. De plus, le(s) réacteur(s) de zone réactionnelle peu(ven)t fonctionner, indépendamment les uns des autres dans le cas de la présence d'au moins deux réacteurs, en courant ascendant ou en courant descendant. Il est possible de combiner les deux modes de circulation lorsque ladite zone comprend au moins deux réacteurs, c'est-à-dire que au moins un réacteur fonctionne en courant ascendant et au moins un réacteur fonctionne en courant descendant. Il est aussi possible d'utiliser au moins un réacteur de type radial.

Les catalyseurs selon l'invention, comprenant les solides catalytiques décrits précédemment, peuvent être fabriqués selon diverses granulométries. Ainsi, la société DEGUSSA propose le produit DELOXAN ASP sous deux formes commerciales :
o diamètre des particules : entre 0,4 et 1,6 mm, pour une application en lit fixe
o diamètres des particules : 0,1 à 0,4 mm pour une mise en oeuvre en suspension, expansion ou fludisée.

Mais toute autre gamme de taille de particules est aussi envisageable dans le cadre de la présente invention, si elle est adaptée à la mise en oeuvre désirée du procédé selon l'invention. De préférence le diamètre moyen équivalent des particules de catalyseur est généralement compris entre 0,1 et 10 mm.

D'autre part, la réaction de décomposition des éthers est fortement endothermique. Elle peut donc donner lieu à des gradients de température importants dans le réacteur, ce qui implique généralement deux inconvénients majeurs :
o une partie du catalyseur ne fonctionne pas dans des conditions thermiques optimales. En effet, une température trop basse limite l'activité catalytique, à la fois d'un point de vue cinétique et d'un point de vue thermodynamique.
o il existe un gradient de sélectivité de la réaction qui peut être difficile à contrôler.

Afin de limiter l'impact de l'endothermicité de la réaction, on propose d'opérer le procédé de l'invention selon diverses mises en oeuvre préférées.

Une des mises en oeuvre préférées du procédé selon l'invention est telle que toute zone réactionnelle comprend au moins un réacteur en lit fixe, fonctionnant en courant ascendant ou descendant, et que ledit réacteur est de préférence équipé de moyen(s) permettant d'apporter des calories à divers endroits à l'intérieur du réacteur. A titre d'illustration non restrictive, on peut citer l'exemple du réacteur multitubulaire, tel que décrit à la page 1311 de l'ouvrage "Le Pétrole, Raffinage et Génie Chimique", tome II, de Pierre Wuithier (Editions TECHNIP). Un des avantages d'une telle mise en oeuvre est que l'apport de calories sur une partie ou toute la longueur du réacteur permet au moins en partie d'homogénéiser la température et ainsi de rattraper le phénomène endothermique. On parle généralement de technique qui permet de travailler le plus possible en "proche isotherme".

Une autre des mises en oeuvre préférées du procédé selon l'invention est telle qu'au moins une, de préférence toute zone réactionnelle comprend au moins deux réacteurs disposés en série et équipés d'au moins un moyen d'échange de chaleur intermédiaire de façon à apporter des calories à l'entrée d'au moins un, de préférence de chaque réacteur et éventuellement également à l'intérieur d'au moins un, de préférence de chaque réacteur (comme indiqué selon la mise en oeuvre préférée précédente).

Une autre des mises en oeuvre préférées du procédé selon l'invention, indépendemment ou non de la mise en oeuvre précédente, est telle que toute zone réactionnelle comprend au moins un réacteur choisi parmi les réacteurs qui fonctionnent en lit mobile, en lit expansé ou en lit fluidisé. Un des avantages d'une telle mise en oeuvre est que ledit réacteur améliore au moins en partie les échanges thermiques et va ainsi dans le sens d'une homogénéisation de la température (c'est-à-dire d'une diminution du gradient de température et donc d'une optimisation du fonctionnement du catalyseur). Une première variante d'une telle mise en oeuvre est telle que ledit réacteur comprend au moins un moyen de recirculation (autour du (ou des) réacteur(s) concerné(s)). Une seconde variante d'une telle mise en oeuvre est telle que la forme géométrique est appropriée, c'est-à-dire telle que la vitesse linéaire puisse être importante à l'intérieur du réacteur ; en pratique cela se traduit par exemple par un faible diamètre de réacteur. Une combinaison des deux variantes citées précédemment peut également être mise en oeuvre. Un des avantages d'une telle mise en oeuvre avec recirculation est une grande souplesse vis-à-vis de la charge à traiter : le temps de séjour (ou la vitesse linéaire) dans la zone réactionnelle peut être maintenue stable malgré des variations de débit de la charge à traiter. Par suite, une telle mise en oeuvre comporte au moins deux avantages, qui sont une facilité d'opération du procédé et un meilleur contrôle des réactions secondaires, grâce à la totale maîtrise de la VVH, c'est-à-dire une optimisation des rendements tant en oléfine tertiaire qu'en alcool.

Une autre des mises en oeuvre préférées du procédé selon l'invention, indépendamment ou non des mises en oeuvre précédentes, est telle que, au moins une, de préférence toute zone réactionnelle comprend au moins deux, de préférence de 2 à 10, réacteurs en parallèle, possédant de préférence des systèmes de chauffage indépendants. Un des avantages d'une telle mise en oeuvre est que le phénomène endothermique est alors réparti dans au moins deux réacteurs ce qui conduit à l'obtention de réacteurs dans lesquels, et pour chacun d'entres eux, les gradients de température sont plus faibles. Dans ladite mise en oeuvre, il est préférable, sans que cela soit restrictif, de travailler en lit fixe dans chacun des réacteurs en parallèle, en circulation ascendante ou descendante. Un autre des avantages d'une telle mise en oeuvre est une très grande souplesse vis-à-vis de la charge à traiter ; selon la quantité de charge à traiter, il est possible d'alimenter la totalité ou seulement une partie (ou un certain nombre) des réacteurs en parallèle. Le temps de séjour dans le réacteur peut ainsi être maintenu stable malgré des variations de débit de la charge à traiter. Par suite, une telle mise en oeuvre comporte au moins deux avantages, qui sont une facilité d'opération du procédé et un meilleur contrôle des réactions secondaires, c'est-à-dire une optimisation des rendements tant en oléfine tertiaire qu'en alcool.

Quelle que soit la mise en oeuvre du procédé selon l'invention, le procédé peut comporter au moins un recyclage d'au moins une partie de l'effluent de la zone réactionnelle dans ladite zone réactionnelle, de façon à réintroduire dans ladite zone au moins une partie de l'éther qui n'a pas réagi, après purification (c'est-à-dire élimination de la majeure partie des produits de la réaction, c'est-à-dire oléfine(s) tertiaire(s) et alcool(s), et des produits secondaires éventuels, tels que des dimères).

De plus, quelle que soit la mise en oeuvre du procédé selon l'invention, au moins une partie de l'effluent de la zone réactionnelle peut être directement incorporée aux fractions essence, après élimination de la majeure partie des principaux produits de la réaction (c'est-à-dire oléfine(s) tertiaire(s) et alcool(s).

Dans ce cas, l'éther qui n'a pas réagi est incorporé aux fractions essences sans être nécessairement débarrassé de la majeure partie des oligomères et autres sous-produits de la réaction. Il en résulte alors un gain au niveau du coût économique du procédé selon l'invention

Une autre des mises en oeuvre préférées du procédé selon l'invention, indépendamment ou non des mises en oeuvre précédentes, est telle que l'on opère à conversion limitée dans au moins une zone réactionnelle, puis que l'on réalise une réaction de finition dans une zone réactionnelle, qui peut être limitée à un seul réacteur isolé en aval.

Les exemples suivants illustrent l'invention.

### Exemple 1

3 g de catalyseur DELOXAN ASP de granulométrie comprise entre 0,4 et 1,6 mm sont introduits dans un réacteur tubulaire possédant un volume utile de 10 ml. Le réacteur est muni d'une double enveloppe où circule un fluide caloporteur qui assure la régulation de température. Un thermocouple mobile au sein d'une gaine traverse le réacteur de part en part ; il permet de connaître le gradient de température dans le réacteur. Le réacteur est maintenu sous pression au moyen d'un détendeur taré à 7.10⁵ Pa relatif. L'installation est équipée d'un chromatographe en ligne de type FID qui permet d'analyser directement la totalité de l'effluent. On alimente le réacteur par une charge contenant plus de 96 % poids de MTBE. Les résultats obtenus sont donnés dans le tableau 1.

Cet exemple illustre l'importante activité du catalyseur qui permet de travailler avec des VVH élevées, minimisant ainsi les réactions secondaires qui dégradent les sélectivités en isobutène et en méthanol.

**Tableau 1**

| Température du fluide caloporteur (°C) | VVH (h-1) | Température moyenne dans le réacteur (°C) | Conversion du MTBE (%) | Sélectivité en isobutène (%) | Sélectivité en méthanol(%) |
|---|---|---|---|---|---|
| 130 | 4,3 | 121,1 | 63 | 96,7 | 98,8 |
| 160 | 24 | 121,0 | 64,5 | 99,7 | 99,9 |

### Exemple 2

1 g de catalyseur DELOXAN ASP de granulométrie comprise entre 0,4 et 1,6 mm est introduit dans un réacteur tubulaire possédant un volume utile de 10 ml. Le réacteur est muni d'une double enveloppe où circule un fluide caloporteur qui assure la régulation de température. Un thermocouple mobile au sein d'une gaine traverse le réacteur de part en part ; il permet de connaître le gradient de température dans le réacteur. Le réacteur est maintenu sous pression au moyen d'un détendeur taré à 7.10⁵ Pa relatif. L'installation est équipée d'un chromatographe en ligne de type FID qui permet d'analyser directement la totalité de l'effluent. On alimente le réacteur par une charge contenant plus de 96 % poids de MTBE. Les résultats obtenus.sont donnés dans le tableau 2.

Cet exemple illustre, en plus de l'exceptionnelle activité du catalyseur, l'ampleur du phénomène endothermique, notamment lorsque l'on travaille à forte VVH.

**Tableau 2**

| Age du catalyseur (h) | Température du fluide caloporteur T1 (°C) | VVH (h-1) | Température moyenne dans le réacteur T2 (°C) | ΔT moyen (T1-T2) (°C) | Conversion du MTBE (%) |
|---|---|---|---|---|---|
| 85 | 160 | 6 | 148,1 | 11,9 | 83,2 |
| 110 | 180 | 6 | 165,7 | 14,3 | 91,6 |
| 190 | 180 | 13,5 | 149,2 | 30,8 | 88 |
| 220 | 180 | 23,5 | 143,5 | 36,5 | 74 |
| 245 | 200 | 8 | 178,9 | 21,1 | 95,4 |
| 270 | 200 | 13,5 | 163,5 | 36,5 | 92,6 |
| 310 | 200 | 23,5 | 158 | 42 | 82 |
| 390 | 220 | 8,5 | 200,9 | 19,1 | 97,5 |
| 420 | 220 | 13,5 | 182,8 | 37,2 | 96,5 |
| 450 | 220 | 23,5 | 174,4 | 45,6 | 92 |

### Exemple 3

Cet exemple illustre l'activité mais aussi la stabilité du catalyseur. L'appareillage et la quantité de catalyseur sont les mêmes que dans le cas de l'exemple 1. Les résultats sont présentés dans le tableau 3.

**Tableau 3**

| Age du catalyseur (h) | Température du fluide caloporteur (°C) | VVH (h-1) | Conversion du MTBE (%) |
|---|---|---|---|
| 250 | 130 | 4,3 | 62 |
| 450 | 130 | 4,3 | 62 |
| 700 | 130 | 4,3 | 61 |
| 930 | 130 | 4,3 | 60,5 |
| 1010 | 180 | 4,3 | 93,4 |
| 1100 | 180 | 4,3 | 92,8 |
| 1250 | 180 | 4,3 | 92,5 |
| 1500 | 180 | 4,3 | 92,1 |
| 1750 | 180 | 4,3 | 92,0 |
| 2100 | 180 | 4,3 | 91,8 |

### Exemple 4 (comparatif)

Cet exemple met en oeuvre un catalyseur connu de l'art antérieur, tel que décrit et revendiqué par le brevet US 5 171 920. A la fois l'activité et la stabilité du catalyseur, mis en oeuvre ici dans les mêmes conditions que celles de l'exemple 3, sont beaucoup moins bonnes que celles présentées dans l'exemple 3.

Mise à part la taille du réacteur, l'appareillage est le même que dans le cas des exemples 1, 2 et 3.

67,2 g de catalyseur de type MgO/SiO₂, préparé selon l'invention décrite dans le brevet US-A-5.171.920, de granulométrie comprise entre 2 et 3 mm, est introduit dans un réacteur tubulaire possédant un volume utile de 145 ml. On alimente le réacteur par une charge contenant 96 % poids de MTBE. Les résultats obtenus sont donnés dans le tableau 4.

**Tableau 4**

| Age du catalyseur (h) | Température du fluide caloporteur (°C) | VVH (h-1) | Conversion du MTBE (%) |
|---|---|---|---|
| 0 | 200 | 1 | 63 |
| 100 | 200 | 1 | 60 |
| 200 | 200 | 1 | 57 |
| 300 | 200 | 1 | 54 |
| 400 | 200 | 1 | 51 |

## Revendications

1. Procédé de décomposition d'éther(s) alkyl tertiaire(s) pour la production d'oléfine(s) tertiaire(s), mettant en oeuvre un catalyseur comprenant au moins un solide de type polysiloxane greffé par au moins un groupement organique de type acide alkyl sulfonique, aryl sulfonique ou alkyaryl sulfonique, à des conditions opératoires telles que la pression (relative) est comprise entre 1 et 10.10⁵ Pa, la température est comprise entre 100 et 250°C, et la VVH (vitesse spatiale liquide horaire, exprimée en volume de charge liquide par volume de catalyseur et par heure) est comprise entre 4,3 et 25 h⁻¹.

2. Procédé selon la revendication 1 tel que le catalyseur est mis en oeuvre dans au moins une zone réactionnelle comprenant au moins un réacteur fonctionnant en lit fixe, en lit mobile, en lit expansé ou encore en lit fluidisé.

3. Procédé selon la revendication 2 tel que le catalyseur est mis en oeuvre dans au moins une zone réactionnelle comprenant au moins un réacteur qui fonctionne en courant ascendant ou en courant descendant

4. Procédé selon la revendication 3 tel que ledit réacteur est équipé de moyen(s) permettant d'apporter des calories à divers endroits à l'intérieur du réacteur.

5. Procédé selon l'une des revendications 3 ou 4 tel que ledit réacteur est multitubilaire.

6. Procédé selon l'une des revendications 3 à 5 tel que au moins une zone réactionnelle comprend deux réacteurs disposés en série et équipés d'au moins un moyen d'échange de chaleur intermédiaire de façon à apporter des calories à l'entrée d'au moins un réacteur.

7. Procédé selon la revendication 6 tel que lesdits réacteurs sont équipés d'au moins un moyen d'échange de chaleur intermédiaire de façon à apporter également des calories à l'intérieur d'au moins un réacteur.

8. Procédé selon la revendication 1 tel que au moins une des zones réactionnelles comprend au moins un réacteur fonctionnant en lit mobile, en lit expansé ou en lit fluidisé.

9. Procédé selon la revendication 8 tel que ledit réacteur comprend au moins un moyen de recirculation ou tel que ledit réacteur a une forme géométrique appropriée au mode de fonctionnement en lit mobile, en lit expansé ou en lit fluidisé.

10. Procédé selon l'une des revendications 2, 8 ou 9 tel que au moins une zone réactionnelle comprend au moins deux réacteurs en parallèle.

11. Procédé selon la revendication 10 tel que la zone réactionnelle comprend de 2 à 10 réacteurs en parallèle.

12. Procédé selon l'une des revendications 10 ou 11 tel que les réacteurs en parallèle possèdent des systèmes de chauffage indépendants.

13. Procédé selon l'une des revendications 10 à 12 tel que l'on alimente une partie desdits réacteurs.

14. Procédé selon l'une des revendications 1 à 13 comportant au moins un recyclage d'au moins une partie de l'effluent de l'une des zones réactionnelles dans ladite zone réactionnelle.

15. Procédé selon l'une des revendications 1 à 14 tel que au moins une partie de l'effluent de l'une des zones réactionnelles est directement incorporée aux fractions essence.

16. Procédé selon la revendication 15 tel que ladite incorporation se fait après purification de la majeure partie de ladite partie dudit effluent.

17. Procédé selon l'une des revendications 1 à 16 tel que le solide minéral greffé par au moins un groupement organique acide alkyl sulfonique compris dans le catalyseur utilisé dans le procédé selon l'invention comprend au moins une unité de formule (1) suivante :
(O_{3/2}Si―R¹―SO₃-)H⁺ (1),
Où R¹ est un alkyl, un aryl ou un alkylaryl.

18. Procédé selon l'une des revendications 1 à 17 de décomposition d'éther(s) alkyl tertiaire(s) choisi(s) dans le groupe formé par l'ETBE le MTBE pour la production d'isobutène ou dans le groupe formé par le TAME et ETAE pour la formation d'isoamylènes.

## Patentansprüche

1. Verfahren zur Zersetzung von (einem) tertiären Alkylether(n) zur Herstellung von (einem) tertiären Olefin(en) unter Einsatz eines Katalysators, der wenigstens einen Festkörper vom Typ von durch wenigstens eine organischen Gruppe vom Typ Alkylsulfonsäure, Arylsulfonsäure oder Alkyl-Arylsulfonsäure gepfropften Siloxans umfasst, bei solchen Betriebsbedingungen, dass der relative Druck zwischen 1 und 10.10⁵ Pa liegt, die Temperatur zwischen 100 und 250°C liegt und die VVH (stündliche Flüssigraumgeschwindigkeit ausgedrückt in Chargenflüssigkeitsvolumen pro Katalysatorvolumen und pro Stunde) bis 4,3 und 25h⁻¹.

2. Verfahren nach Anspruch 1, derart, dass der Katalysator in wenigstens einer Reaktionszone eingesetzt wird, die wenigstens einen Reaktor umfasst, der im Festbett, mobilen Bett, expandierten Bett oder auch im fluidisierten Bett arbeitet.

3. Verfahren nach Anspruch 2, derart, dass der Katalysator in wenigstens einer Reaktionszone eingesetzt wird, die wenigstens einen Reaktor umfasst, der bei aufsteigendem Strom oder bei absteigendem Strom arbeitet.

4. Verfahren nach Anspruch 3, derart, dass der Reaktor mit (einem) Mittel(n) ausgerüstet ist, dass (die) es ermöglicht (ermöglichen) Wärme zu verschiedenen Stelle in den Reaktor zu bringen.

5. Verfahren nach einem der Ansprüche 3 oder 4, derart, der Reaktor multitubular ist.

6. Verfahren nach einem der Ansprüche 3 bis 5, derart, dass wenigstens eine Reaktionszone zwei Reaktor umfasst, die in Reihe angeordnet sind und wenigstens einem Mittel zum intermediären Wärmeaustausch derart ausgerüstet sind, um Wärme zum Eingang wenigstens eines Reaktors zu bringen.

7. Verfahren nach Anspruch 6, derart, dass die Reaktoren mit wenigstens einem Mittel zum intermediären Wärmeaustausch derart ausgerüstet sind, um gleichermaßen Wärme ins Innere wenigstens eines Reaktors zu bringen.

8. Verfahren nach Anspruch 1, derart, dass wenigstens eine der Reaktionszonen wenigstens einen Reaktor umfasst, der im mobilen Bett, im expandierten Bett oder im fluidisierten Bett arbeitet.

9. Verfahren nach Anspruch 8, derart, dass der Reaktor wenigstens ein Mittel zur Rezirkulierung umfasst oder derart, dass der Reaktor eine für den Betriebesmodus im mobilen Bett, expandierten Bett oder fluidisierten Bett geeignete geometrische Form hat.

10. Verfahren nach einem der Ansprüche 2, 8 oder 9, derart, dass wenigstens eine Reaktionszone wenigstens zwei parallele Reaktoren umfasst.

11. Verfahren nach Anspruch 10, derart, dass die Reaktionszone 2 bis 10 parallele Reaktoren umfasst.

12. Verfahren nach einem der Ansprüche 10 oder 11, derart, dass die parallelen Reaktoren unabhängige Heizsysteme aufweisen.

13. Verfahren nach einem der Ansprüche 10 bis 12, derart, dass man einen Teil der Reaktoren versorgt.

14. Verfahren nach einem der Ansprüche 1 bis 13, dass wenigstens eine Reziklierung in wenigstens einem Teil des Abstroms von wenigstens einer Reaktionszone in die Reaktionszone umfasst.

15. Verfahren nach einem der Ansprüche 1 bis 14, derart, dass wenigstens ein Teil des Abstroms von einer der Reaktionszonen direkt mit den Benzinfraktionen vereinigt wird.

16. Verfahren nach Anspruch 15, derart, dass diese Vereinigung nach Reinigung des größeren Teils dieses Teils dieses Abstroms stattfindet.

17. Verfahren nach einem der Ansprüche 1 bis 16, derart, dass die mineralische Festkörper der durch wenigstens eine organische Alkylsulfonsäuregruppe gepropft ist und in dem Katalysator enthalten ist, der bei dem Verfahren der vorliegenden Erfindung verwendet wird, wenigstens eine Einheit der folgenden Formel 1 umfaßt:
O₃/₂Si―R¹―SO₃-)H⁺ (1),
wobei R¹ ein Alkyl, ein Aryl oder als Alkylaryl ist.

18. Verfahren nach einem der Ansprüche 1 bis 17 zur Zersetzung von (einem) tertiären Alkylether(n) gewählt aus der Gruppe, die gebildet wird durch ETBE, MTBE für die Herstellung von Isobuten oder aus der Gruppe, die gebildet ist durch TAME und ETAE für die Bildung von Isamylenen.

## Claims

1. A process for the decomposition of tertiary alkyl ether(s) for the production of tertiary olefin(s), using a catalyst comprising at least a polysiloxane type solid grafted with at least one alkyl sulphonic acid, aryl sulphonic acid or alkylaryl sulphonic acid type organic group at operating conditions such that the (relative) pressure is in the range 1 to 10.10⁵Pa, the temperature is in the range 100 to 250°C and the HSV is in the range 4.3 to 25 h⁻¹.

2. A process according to claim 1, in which the catalyst is used in at least one reaction zone comprising at least one reactor functioning as a fixed bed, mobile bed, expanded bed or a fluidised bed.

3. A process according to claim 2, in which the catalyst is used in at least one reaction zone comprising at least one reactor which functions in up flow or in down flow mode.

4. A process according to claim 3, in which said reactor is provided with means for adding heat to various regions inside the reactor.

5. A process according to one of claims 3 to 4 in which said reactor is a multitubular reactor.

6. A process according to one of claims 3 to 5, in which at least one reaction zone comprises two reactors disposed in series and provided with at least one intermediate heat exchange means for providing heat to the inlet to at least one reactor.

7. A process according to claim 6, in which said reactors are provided with at least one intermediate heat exchange means for providing heat to the inside of at least one reactor.

8. A process according to claim 1, in which at least one reaction zone comprises at least one reactor operating as a mobile bed, and expanded bed or a fluidised bed.

9. A process according to claim 8, in which said reactor comprises at least one recirculation means or such that each reactor has a geometric shape which is appropriate for operating as a mobile bed, expanded bed or fluidised bed.

10. A process according to one of claims 2, 8 or 9, in which at least one reaction zone comprises at least two reactors in parallel.

11. A process according to claim 10, in which the reaction zone comprises 2 to 10 reactors in parallel.

12. A process according to one of claims 10 to 11, in which the reactors in parallel are provided with independent heating means.

13. A process according to one of claims 10 to 12, in which a portion of said reactors is supplied.

14. A process according to one of claims 1 to 13, comprising at least one recycling step for at least a portion of the effluent from one of the reaction zones to said reaction zone.

15. A process according to one of claims 1 to 14, in which at least a portion of the effluent from one of the reaction zones, is directly incorporated into gasoline fractions.

16. A process according to claim 15, in which said incorporation is made after purification of the major portion of said effluent.

17. A process according to one of claims 1 to 16, in which the inorganic solid grafted with at least one alkyl sulphonic acid organic group comprised in the catalyst used in the process of the invention comprises at least one unit with the following formula(1):
(O_{3/2}Si---R¹---SO⁻₃)H⁺ (1),
Where R¹ is an alkyl, aryl or alkylaryl radical.

18. A process according to one of claims 1 to 17 for the decomposition of tertiary alkyl ether(s) selected from the group formed by ETBE and MTBE for the production of isobutene or from the group formed by TAME and ETAE for the formation of isoamylenes.
